(19) Europäisches Patentamt
European Patent Office
Office européen des brevets

(11) **EP 3 429 682 B1**

(12) **EUROPÄISCHE PATENTSCHRIFT**

(45) Veröffentlichungstag und Bekanntmachung des Hinweises auf die Patenterteilung:
**29.01.2020   Patentblatt 2020/05**

(21) Anmeldenummer: **17710870.1**

(22) Anmeldetag: **14.03.2017**

(51) Int Cl.:
*A61N 1/36* *(2006.01)*          *A61N 5/06* *(2006.01)*
*A61B 5/00* *(2006.01)*          *A61N 5/067* *(2006.01)*
*A61B 5/04* *(2006.01)*

(86) Internationale Anmeldenummer:
**PCT/EP2017/055915**

(87) Internationale Veröffentlichungsnummer:
**WO 2017/157890 (21.09.2017 Gazette 2017/38)**

(54) **VORRICHTUNG ZUR EFFEKTIVEN, INVASIVEN UND AMPLITUDENMODULIERTEN NEUROSTIMULATION**

DEVICE FOR EFFECTIVE, INVASIVE AND AMPLITUDE-MODULATED NEUROSTIMULATION

DISPOSITIF DE NEUROSTIMULATION EFFICACE, EFFRACTIVE ET MODULÉE EN AMPLITUDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.03.2016   DE 102016104913**

(43) Veröffentlichungstag der Anmeldung:
**23.01.2019   Patentblatt 2019/04**

(73) Patentinhaber: **Forschungszentrum Jülich GmbH 52425 Jülich (DE)**

(72) Erfinder:
• **TASS, Peter Alexander**
  **83684 Tegernsee (DE)**
• **POPOVYCH, Oleksandr**
  **52355 Düren (DE)**

(74) Vertreter: **Vossius & Partner Patentanwälte Rechtsanwälte mbB Siebertstrasse 3 81675 München (DE)**

(56) Entgegenhaltungen:
**US-A1- 2010 217 355      US-A1- 2011 201 977
US-A1- 2013 090 519      US-A1- 2014 336 547**

## EP 3 429 682 B1

**Beschreibung**

Die Erfindung betrifft eine Vorrichtung zur effektiven, invasiven und amplitudenmodulierten Neurostimulation.

Bei Patienten mit neurologischen oder psychiatrischen Erkrankungen, z. B. Morbus Parkinson, essentiellem Tremor, Dystonie oder Zwangserkrankungen, sind Nervenzellverbände in umschriebenen Bereichen des Gehirns, z. B. des Thalamus und der Basalganglien krankhaft, z. B. übersteigert synchron, aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Beim Morbus Parkinson verändert die pathologisch synchrone Aktivität die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalganglien beispielsweise den Großhirnrindenarealen ihren Rhythmus auf, so dass schließlich die von diesen Arealen gesteuerten Muskeln pathologische Aktivität, z. B. ein rhythmisches Zittern (Tremor), entfalten.

Zur Behandlung von medikamentös nicht hinreichend behandelbaren Parkinsonpatienten wird die tiefe Hirnstimulation eingesetzt. Hierbei werden Tiefenelektroden in speziellen Hirngebieten, z. B. im Nucleus subthalamicus, implantiert. Zur Linderung der Symptome wird über die Tiefenelektroden eine elektrische Reizung durchgeführt. Bei der Standard-Hochfrequenz-Stimulation zur Behandlung der Parkinsonschen Erkrankung wird eine sogenannte Hochfrequenz-Dauerreizung bei Frequenzen von über 100 Hz durchgeführt. Diese Behandlungsart hat keine lang anhaltenden therapeutischen Effekte (vgl. P. Temperli, J. Ghika, J.-G. Villemure, P. Burkhard, J. Bogousslavsky, and F. Vingerhoets: How do parkinsonian signs return after discontinuation of subthalamic DBS? Neurology 60, 78 (2003)). Lang anhaltende therapeutische Effekte können - obendrein mit deutlich weniger Reizung (z. B. Reizstrom) - durch die "Coordinated Reset"-Stimulation (CR-Stimulation) hervorgerufen werden (vgl. P. A. Tass, L. Qin, C. Hauptmann, S. Doveros, E. Bezard, T. Boraud, W. G. Meissner: Coordinated reset neuromodulation has sustained after-effects in parkinsonian monkeys. Annals of Neurology 72, 816-820 (2012); I. Adamchic, C. Hauptmann, U. B. Barnikol, N. Pawelcyk, O.V. Popovych, T. Barnikol, A. Silchenko, J. Volkmann, G. Deuschl, W. Meissner, M. Maarouf, V. Sturm, H.-J. Freund, P. A. Tass: Coordinated Reset Has Lasting Aftereffects in Patients with Parkinson's Disease. Movement Disorders 29, 1679 (2014)).

Bei anderen Erkrankungen, z. B. bei medikamentös nicht ausreichend behandelbaren Epilepsien, werden neben Tiefenelektroden auch andere, z. B. epikortikale oder epidurale Elektroden, implantiert. Bei weiteren Erkrankungen, z. B. chronischen Schmerz-Syndromen, ist es üblich, nicht nur mittels Tiefenelektroden im Gehirn, sondern auch mittels z. B. epiduralen Elektroden das Rückenmark zu reizen. Anders als die CR-Stimulation haben die meisten anderen Stimulationsarten keine lang anhaltenden therapeutischen Effekte.

Therapeutische Effekte können auch durch direkte Stimulation des Hirngewebes bzw. Rückenmarks mit Licht, z. B. über implantierte Lichtleiter, erzielt werden. Hierbei können auch unterschiedliche raum-zeitliche Stimulationsmuster, wie z. B. die CR-Stimulation, zur Anwendung kommen.

Die Wirkung der CR-Stimulation kann durch eine ungünstige Wahl der Stimulationsparameter, insbesondere der CR-Stimulationsfrequenz und der Stimulationsintensität im Sinne der Amplitude der Einzelreize und/oder der Dauer der Einzelreize, deutlich vermindert bzw. sogar unterbunden werden. Falsch bzw. sub-optimal gewählte Parameter können den Stimulationserfolg schwächen oder sogar vollständig unterbinden. Es ist somit wichtig, die Stimulationsparameter zu kalibrieren. Da Parameter des stimulierten Gewebes zeitlichen Schwankungen unterworfen sind, ist eine in hinreichenden zeitlichen Abständen erfolgende Kalibration notwendig. Da derartige Schwankungen in einer nicht vorhersehbaren Weise auftreten können, ist in einem "open loop"-Modus die Kalibration vergleichsweise häufig durchzuführen, und/oder es sind Feedback-Signale, d. h. Rückkopplungssignale, in einem "closed loop"-Modus abzuleiten, welche die Notwendigkeit der Re-Kalibration, z. B. im Sinne des Überschreitens einer tolerablen neuronalen Synchronisation, anzuzeigen vermögen.

Aus diesem Grund wurden Stimulationsmethoden entwickelt, welche mit deutlich weniger Stimulationsparametern als die CR-Stimulation auskommen, z. B. die lineare zeitverzögerte Feedback-Stimulation (vgl. M. G. Rosenblum, A. S. Pikovsky: Controlling synchronization in an ensemble of globally coupled oscillators. Physical Review Letters 92, 114102 (2004)) oder die nicht-lineare zeitverzögerte Feedback-Stimulation (vgl. O. V. Popovych, C. Hauptmann, P. A. Tass: Effective Desynchronization by Nonlinear Delayed Feedback. Physical Review Letters 94, 164102 (2005)), jeweils über einen oder mehrere Stimulationskontakte. Die herkömmliche nicht-lineare zeitverzögerte Feedback-Stimulation ist der herkömmlichen linearen zeitverzögerten Feedback-Stimulation dadurch klar überlegen, dass bei ersterer über weite Bereichen der Zeitverzögerung eine Desynchronisation erzielt werden kann, während bei letzterer nur in engen Bereichen der Zeitverzögerung eine Desynchronisation erzielt werden kann; außerhalb dieser engen Bereiche führt diese Stimulationsmethode zu einer Synchronisation bzw. stabilisiert den synchronen Zustand.

Sowohl die herkömmliche lineare zeitverzögerte Feedback-Stimulation als auch die herkömmliche nicht-lineare zeitverzögerte Feedback-Stimulation sind in ihrer Wirksamkeit stark begrenzt, da bei therapeutisch wirksamen Stimulationsstärken der Ladungseintrag pro Halbschwingung typischerweise die zulässigen Obergrenzen zur Vermeidung von Gewebeschäden deutlich übersteigt (vgl. S. B. Brummer, M. Turner, M.: Electrical stimulation of the nervous system: the principle of safe charge injection with noble metal electrodes. Bioelectrochem. Bioenerg. 2, 13 (1975); S. B. Brummer, L. S. Robblee, F. T. Hambrecht: Criteria for selecting electrodes for electrical stimulation: theoretical and practical considerations. Ann. N. Y. Acad. Sci. 405. 159 (1983); D. Harnack, C. Winter, W. Meissner, T. Reum, A. Kupsch, R. Morgenstern: The effects of electrode material, charge density and stimulation duration on the safety of high-frequency stimulation of the subthalamic nucleus in rats. J. Neurosci. Methods 138, 207 (2004)). Dies liegt daran, dass die lineare zeitverzögerte Feedback-Stimulation das gemessene Signal der zu desynchronisierenden Neuronenpopulation bzw. ein hinreichend eng damit gekoppeltes oszillatorisches Signal nach Verstärkung, Bandpass-Filterung (oder analoger Vorverarbeitung zur Extraktion der relevanten pathologischen Frequenzkomponente) und Zeitverzögerung (typischerweise mit der halben mittleren Periode der synchronisierten Oszillation) als Stimulationssignal verwendet. Durch die hieraus resultierende niedrige Stimulationsfrequenz übersteigt der Ladungseintrag die zulässigen Obergrenzen schon bei vergleichsweise geringen Reizstärken. Bei der nicht-linearen zeitverzögerten Feedback-Stimulation wird durch nichtlineare Verrechnung des zeitverzögerten und nicht zeitverzögerten vorverarbeiteten Signals in gleicher Weise ein Stimulationssignal mit derselben dominanten Frequenz wie bei der linearen zeitverzögerten Feedback-Stimulation erzeugt.

US 2010/0217355 A1 offenbart eine Vorrichtung zur Stimulation von Neuronen gemäß dem Oberbegriff des ersten Anspruchs.

Der Erfindung liegt die Aufgabe zugrunde, eine Vorrichtung zur Stimulation von Neuronen anzugeben, mit der sich im Vergleich zum Stand der Technik die Reizung deutlich weniger fehleranfällig und robuster durchführen und der gewünschte Desynchronisationseffekt ohne aufwändige Kalibration erzielen lässt.

Die der Erfindung zugrunde liegende Aufgabenstellung wird durch die Merkmale der unabhängigen Ansprüche gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1          eine schematische Darstellung einer Vorrichtung zur Desynchronisierung von Neuronen mit einer krankhaft synchronen und oszillatorischen neuronalen Aktivität gemäß einer Ausgestaltung;

Fig. 2          eine schematische Darstellung eines Pulszugs mit Einzelpulsen, die eine Pause zwischen einem ersten Pulsanteil und einem dem ersten Pulsanteil nachfolgenden zweiten Pulsanteil aufweisen;

Fig. 3 und 4   schematische Darstellungen von Pulszügen, deren Amplitude mit verschiedenen Modulationssignalen moduliert wurde;

Fig. 5 und 6   schematische Darstellungen von Vorrichtungen zur Desynchronisierung von Neuronen mit einer krankhaft synchronen und oszillatorischen neuronalen Aktivität mittels elektrischer Stimulationssignale gemäß weiteren Ausgestaltungen;

Fig. 7          eine schematische Darstellung einer Vielkanal-Elektrode;

Fig. 8          eine schematische Darstellung einer Mehrkanal-Elektrode;

Fig. 9          eine schematische Darstellung einer Vielkanal-Elektrode zur direkten Stimulation eines Zielareals und/oder Ableitung von Messsignalen und einer weiteren Vielkanal-Elektrode zur indirekten Stimulation des Zielareals;

Fig. 10 bis 13 Diagramme mit Simulationsergebnissen für eine Hochfrequenz-Dauerstimulation mit einer nicht-linearen zeitverzögerten Feedback-Amplitudenmodulation und Einzelpulsen mit und ohne Pausen zwischen aufeinanderfolgenden Pulsanteilen; und

Fig. 14 bis 16 Diagramme mit Simulationsergebnissen für eine Hochfrequenz-Dauerstimulation mit einer linearen zeitverzögerten Feedback-Amplitudenmodulation und Einzelpulsen mit und ohne Pausen zwischen aufeinanderfolgenden Pulsanteilen.

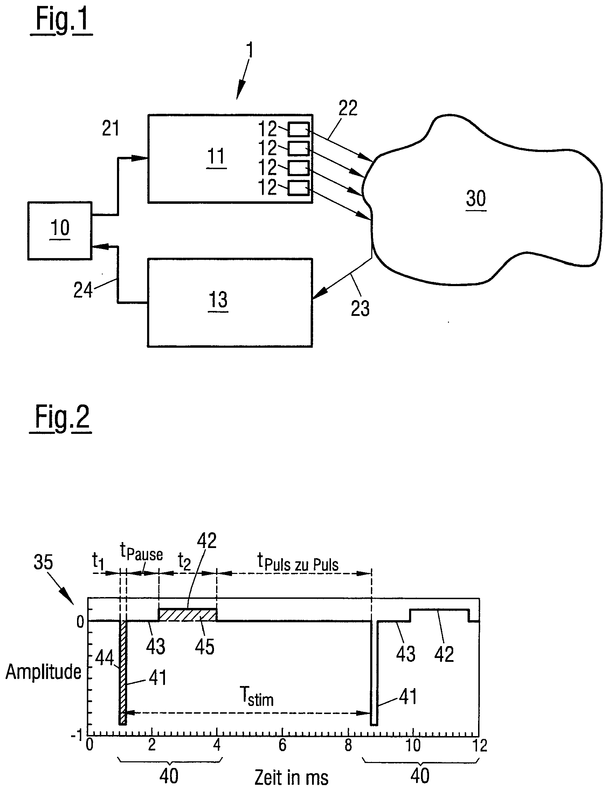

In Fig. 1 ist schematisch eine Vorrichtung 1 zur Stimulation von Neuronen mit einer krankhaft synchronen und oszillatorischen neuronalen Aktivität dargestellt. Die Vorrichtung 1 besteht aus einer Steuereinheit 10, einer Stimulationseinheit 11 mit einem oder mehreren Stimulationselementen 12 und einer Messeinheit 13.

In der in Fig. 1 dargestellten Ausführungsform enthält die Stimulationseinheit 11 beispielhaft vier Stimulationselemente 12. Die Stimulationseinheit 11 kann selbstverständlich aber auch eine andere Zahl von Stimulationselementen 12 aufweisen. Im Fall von elektrischer Stimulation kann es sich bei den Stimulationselementen 12 z. B. um Stimulationskontaktflächen einer oder mehrerer Elektroden zur Applikation elektrischer Reize an das neuronale Gewebe handeln. Falls optisch stimuliert wird, können z. B. Lichtleiter als Stimulationselemente 12 eingesetzt werden, um das neuronale Gewebe an den gewünschten Stellen mit Lichtreizen zu stimulieren.

Die Steuereinheit 10 ist mit der Stimulationseinheit 11 und der Messeinheit 13 gekoppelt und führt während des Betriebs der Vorrichtung 1 eine Steuerung der Stimulationseinheit 11 durch. Dazu erzeugt die Steuereinheit 10 Steuersignale 21, die von der Stimulationseinheit 11 entgegengenommen werden.

Die Stimulationseinheit 11 wird operativ in den Körper des Patienten implantiert und erzeugt anhand der Steuersignale 21 ein oder mehrere Stimulationssignale bzw. Reize 22, insbesondere elektrische und/oder optische Stimulationssignale 22, die an das Gewebe des Patienten appliziert werden, um damit Neuronen in einem Zielareal 30 im Gehirn und/oder Rückenmark des Patienten zu stimulieren. Die Stimulationssignale 22 sind insbesondere dazu ausgelegt, bei einer Verabreichung an den Patienten die Neuronen mit der krankhaft synchronen und oszillatorischen Aktivität zu desynchronisieren.

Die Messeinheit 13 nimmt ein oder mehrere am Patienten gemessene Messsignale 23 auf, wandelt diese gegebenenfalls in elektrische Signale 24 um und führt sie der Steuereinheit 10 zu. Insbesondere kann mittels der Messeinheit 13 die neuronale Aktivität in dem stimulierten Zielareal 30 oder einem mit dem Zielareal 30 verbundenen Gebiet gemessen werden, wobei die neuronale Aktivität dieses Gebiets mit der neuronalen Aktivität des Zielgebiets 30 hinreichend eng korreliert. Mittels der Messeinheit 13 kann auch eine nicht-neuronale, z. B. muskuläre Aktivität oder die Aktivierung des autonomen Nervensystems, gemessen werden, sofern diese mit der neuronalen Aktivität des Zielgebiets 30 hinreichend eng korreliert sind. Weiterhin kann der durch die Stimulationssignale 22 erzielte Stimulationseffekt mit Hilfe der Messeinheit 13 überwacht werden.

Die Messeinheit 13 enthält einen oder mehrere Sensoren, die es insbesondere ermöglichen, die Amplitude der pathologischen oszillatorischen neuronalen Aktivität aufzunehmen.

Die Sensoren können in den Körper des Patienten implantiert sein. Als invasive Sensoren können beispielsweise epikortikale Elektroden, Tiefenhirnelektroden zur Messung von z. B. lokalen Feldpotentialen, sub- oder epidurale Hirnelektroden, subkutane EEG-Elektroden und sub- oder epidurale Rückenmarkselektroden dienen. Die Tiefenelektroden zur Messung der lokalen Feldpotentiale können auch baulich vereint oder sogar identisch mit den für die Stimulation verwendeten Elektroden sein. Die Kontakte der Elektroden können derart platziert werden, dass sie relevante neuronale Feedback-Signale ableiten können.

Alternativ können nicht-invasive Sensoren eingesetzt werden, z. B. chronisch oder intermittent genutzte Elektroenzephalographie (EEG)- oder Elektromyographie (EMG)-Elektroden oder Magnetenzephalographie (MEG)-Sensoren. Die neuronale Aktivität kann auch durch Detektion charakteristischer Bewegungsmuster wie Tremor, Akinese oder epileptische Anfälle mit Hilfe eines Akzelerometers oder Gyroskops oder indirekt durch Messung der Aktivierung des autonomen Nervensystems mittels Messung des Hautleitwiderstands ermittelt werden. Im Falle von LFP-, EEG- und/oder MEG-Signalen können mittels dem Fachmann bekannten Inversmethoden die zugrunde liegenden Ströme berechnet und als die weiter unten beschriebenen Feedback-Modulationssignale verwendet werden.

Die Steuereinheit 10 verarbeitet die Signale 24, z. B. können die Signale 24 verstärkt und/oder gefiltert werden. Ferner erzeugt die Steuereinheit 10 aus den Signalen 24 und damit aus dem Messsignal 23 ein Modulationssignal, mit dem die Amplitude eines eine Vielzahl von Einzelpulsen umfassenden Pulszugs moduliert wird. Die Steuereinheit 10 steuert die Stimulationseinheit 11 derart an, dass das mindestens eine Stimulationselement 12 den amplitudenmodulierten Pulszug als das Stimulationssignal 22 dem Gewebe verabreicht, um damit die Neuronen in dem Zielgebiet 30 zu stimulieren. Die Einzelpulse des Pulszugs bestehen jeweils aus einem ersten Pulsanteil und einem dem ersten Pulsanteil nachfolgenden zweiten Pulsanteil. Einer von dem ersten Pulsanteil und dem zweiten Pulsanteil bringt Ladung in das Gewebe ein und der andere Pulsanteil entnimmt Ladung aus dem Gewebe. Ferner wird zwischen dem ersten Pulsanteil und dem zweiten Pulsanteil der Einzelpulse jeweils eine Pause eingehalten.

Die Steuereinheit 10 kann eine nicht-invasive Einheit sein, d. h., während des Betriebs der Vorrichtung 1 befindet sie sich außerhalb des Körpers des Patienten und wird nicht operativ in den Körper des Patienten implantiert.

Die einzelnen Komponenten der Vorrichtung 1, insbesondere die Steuereinheit 10, die Stimulationseinheit 11 und/oder die Messeinheit 13, können baulich voneinander getrennt sein. Die Vorrichtung 1 kann daher auch als System aufgefasst werden. Zur Durchführung ihrer Aufgaben kann die Steuereinheit 10 einen Prozessor, z. B. einen Mikrocontroller, enthalten. Die hierin beschriebenen Stimulationsverfahren können als Software-Code in einem der Steuereinheit 10 zugeordneten Speicher abgelegt sein.

Die Vorrichtung 1 kann insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bildet eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

In Fig. 2 ist beispielhaft ein Ausschnitt eines Pulszugs 35 dargestellt, aus dem ein Stimulationssignal bzw. Reiz 22 zur Stimulation der Neuronen in dem Zielgebiet 30 generiert werden kann. Fig. 2 zeigt den Pulszug 35 vor der Durchführung der Amplitudenmodulation.

Der Pulszug 35 besteht aus einer Vielzahl von Einzelpulsen 40, die sich insbesondere periodisch wiederholen und von denen in Fig. 2 beispielhaft zwei Einzelpulse 40 dargestellt sind. In Fig. 2 ist die Amplitude bzw. die Signalstärke der Einzelpulse 40, z. B. in normierten Einheiten, gegen die Zeit in ms aufgetragen.

Jeder der Einzelpulse 40 besteht aus einem ersten Pulsanteil 41, einem dem ersten Pulsanteil 41 nachfolgenden zweiten Pulsanteil 42 und einer zwischen dem ersten Pulsanteil 41 und dem zweiten Pulsanteil 42 liegenden Pause 43. Der erste Pulsanteil 41 hat eine Dauer $t_1$, der zweite Pulsanteil 42 hat eine Dauer $t_2$ und die Pause hat eine Dauer $t_{Pause}$.

Der erste und der zweite Pulsanteil 41, 42 sind derart ausgestaltet, dass einer von den beiden Pulsanteilen 41, 42 Ladung in das Gewebe einbringt und der andere Pulsanteil Ladung dem Gewebe entnimmt. In der in Fig. 2 dargestellten Ausführungsform bringt der erste Pulsanteil 41 Ladung in das Gewebe ein und der zweite Pulsanteil 42 entnimmt dem Gewebe Ladung. Alternativ kann vorgesehen sein, dass der erste Pulsanteil 41 Ladung dem Gewebe entnimmt und der zweite Pulsanteil 42 Ladung an das Gewebe abgibt.

Der Betrag der Amplitude des ersten Pulsanteils 41 ist größer als der Betrag der Amplitude des zweiten Pulsanteils 42. Dafür ist die Dauer $t_2$ des zweiten Pulsanteils 42 länger als die Dauer $t_1$ des ersten Pulsanteils 41. Die beiden Pulsanteile 41, 42 sind idealerweise so dimensioniert, dass die Ladung, die durch sie übertragen wird, bei beiden Pulsanteilen 41, 42 gleich groß ist, d. h., die in Fig. 2 schraffiert eingezeichneten Flächen 44 und 45, die durch Integration der Pulsanteile 41 bzw. 42 über die Zeit gewonnen werden, sind gleich groß. Im Ergebnis wird durch einen Einzelpuls 40 genauso viel Ladung während der eigentlichen Stimulationsphase der Dauer $t_1$ in das Gewebe eingebracht, wie dem Gewebe während der ladungsbalancierenden Stimulationsphase der Dauer $t_2$ entnommen wird. Derartige Pulse werden als ladungsbalancierte Pulse bezeichnet.

Die Dauer $t_1$ des ersten Pulsanteils 41 liegt insbesondere im Bereich zwischen 1 $\mu$s und 450 $\mu$s. Sofern es sich um eine elektrische Stimulation handelt, können die Einzelpulse 40 strom- oder spannungskontrollierte Pulse sein. Die Amplitude des ersten Pulsanteils 41 kann im Fall von stromkontrollierten Pulsen bis zu 25 mA und im Fall von spannungskontrollierten Pulsen bis zu 16 V betragen.

Während der zwischen dem ersten Pulsanteil 41 und dem zweiten Pulsanteil 42 liegenden Pause 43 ist die Amplitude des Einzelpulses 40 gleich Null, d. h., während der Pause 43 wird weder Ladung in das Gewebe eingebracht noch wird dem Gewebe Ladung entnommen.

Die Einzelpulse 40 des Pulszugs 35 sind insbesondere identisch und werden periodisch mit einer Frequenz $f_{stim}$ appliziert. Die Frequenz $f_{stim}$, mit welcher die Einzelpulse 40 innerhalb des Pulszugs 35 repetitiv appliziert werden, beträgt insbesondere mindestens 100 Hz, beispielsweise liegt die Frequenz $f_{stim}$ im Bereich von 100 bis 200 Hz. Die Frequenz $f_{stim}$ kann aber auch noch höhere Werte annehmen. In Fig. 2 ist die Periode $T_{stim} = 1/f_{stim}$ dargestellt. Die dauerhafte Applikation eines Pulszugs 35 mit einer Frequenz $f_{stim}$ von mindestens 100 Hz wird als Hochfrequenz-Dauerstimulation bezeichnet.

Während der Zeit $t_{Puls-zu-Puls}$ zwischen zwei innerhalb des Pulszugs 35 direkt aufeinanderfolgenden Einzelpulsen 40, d. h., zwischen dem Ende des zweiten Pulsanteils 42 eines Einzelpulses 40 und dem Beginn des ersten Pulsanteils 41 des direkt nachfolgenden Einzelpulses 40, wird nicht stimuliert, d. h., die Amplitude des Pulszugs 35 beträgt während der Zeit $t_{Puls-zu-Puls}$ Null. Es kann vorgesehen sein, dass die Zeit $t_{Puls-zu-Puls}$ zwischen zwei direkt aufeinanderfolgenden Einzelpulsen 40 länger ist als die Dauer $t_{Pause}$ der Pause 43 zwischen dem ersten und dem zweiten Pulsanteil 41, 42 eines Einzelpulses 40. Es wird daraufhin gewiesen, dass sich die Zeit $t_{Puls-zu-Puls}$ folgendermaßen berechnet:

$$t_{Puls-zu-Puls} = T_{stim} - t_1 - t_2 - t_{Pause} \qquad (1)$$

Wie weiter unten erläutert wird, trägt die Pause 43 zwischen den beiden Pulsanteilen 41, 42 eines Einzelpulses 40 wesentlich zum Stimulationserfolg bei. Gemäß einer Ausgestaltung beträgt die Dauer $t_{Pause}$ der Pause 43 mindestens 1 ms. Gemäß einer weiteren Ausgestaltung liegt die Dauer $t_{Pause}$ der Pause 43 im Bereich von 1 ms bis 6 ms. Weiterhin kann vorgesehen sein, dass die Dauer $t_{Pause}$ der Pause 43 angepasst ist an die Frequenz $f_{stim}$, mit der die Einzelpulse 40 innerhalb des Pulszugs 35 repetitiv appliziert werden. Je größer die Frequenz $f_{stim}$ ist, desto kürzer ist die Perioden-länge $T_{stim}$. Somit wird die maximal mögliche Dauer der Pause 43 unter der Nebenbedingung, dass $t_{Pause} < t_{Puls-zu-Puls}$ gilt, desto kleiner, je größer die Frequenz $f_{stim}$ wird.

Weiterhin kann die Steuereinheit 10 die Dauer $t_{Pause}$ der Pause 43 variieren, bis die Synchronisation der stimulierten Neuronen minimal ist oder einen vorgegebenen Schwellwert unterschreitet.

Der Pulszug 35 mit den periodisch auftretenden Einzelpulsen 40 wird vorzugsweise dauerhaft appliziert, d. h. während eines vergleichsweise langen Zeitraums. Z. B. wird der Pulszug 35 länger als 30 Minuten oder 1 Stunde oder 2 Stunden appliziert. Während der Applikation des Pulszugs 35 werden außer den oben beschriebenen Pausen mit den Längen $t_{Pause}$ und $t_{Puls-zu-Puls}$ vorzugsweise keine weiteren Pausen eingehalten.

Die in Fig. 2 dargestellte Rechteckform der Einzelpulse 40 und insbesondere der ersten und zweiten Pulsanteile 41, 42 stellt eine ideale Form dar. Je nach der Güte der die Einzelpulse 40 erzeugenden Elektronik wird von der idealen Rechteckform abgewichen.

Die in Fig. 2 dargestellten Einzelpulse 40 mit den ersten und zweiten Pulsanteilen 41, 42 können auch als Einzelreize 40 mit ersten und zweiten Reizanteilen 41, 42, die innerhalb einer periodischen Reizfolge appliziert werden, bezeichnet werden. Ferner kann anstelle von ersten und zweiten Pulsanteilen von ersten und zweiten Phasen gesprochen werden.

Die Amplitude des Pulszugs 35 wird wie oben beschrieben mit einem Modulationssignal moduliert, welches die Steuereinheit 10 aus dem Messsignal 23 erzeugt. Da das Messsignal 23 die pathologisch synchrone neuronale Aktivität der stimulierten Neuronen wiedergibt, wird folglich eine Feedback-Amplitudenmodulation durchgeführt, d. h. eine Amplitudenmodulation mit einem Rückkopplungssignal als Modulationssignal. Das Feedback-Modulationssignal, das im Folgenden mit S(t) bezeichnet wird, ist zudem gegenüber dem von der Messeinheit 13 aufgenommenen Messsignal 23 zeitverzögert und linear oder nicht-linear verarbeitet.

Zur Erzeugung des Feedback-Modulationssignals S(t) wird das Messsignal 23 zunächst vorverarbeitet, z. B. verstärkt und/oder bandpassgefiltert, wobei der physiologisch relevante Frequenzbereich von dem Bandpassfilter durchgelassen wird. Ein zu einem Zeitpunkt t aufgenommenes vorverarbeitetes Messsignal 23 soll im Folgenden mit x(t) bezeichnet werden. Ferner ist I der Parameter der Stimulationsintensität, und $\tau$ gibt die Zeitverzögerung des Feedback-Modulationssignals S(t) gegenüber dem Messsignal 23 bzw. dem vorverarbeiteten Messsignal x(t) an. Dann gilt für das lineare zeitverzögerte Feedback-Modulationssignal S(t):

$$S(t) = I\big(x(t-\tau) - x(t)\big) \qquad\qquad (2)$$

Mit dem Modulationssignal S(t) wird die Amplitude des in Fig. 2 gezeigten Pulszugs 35 moduliert, um das Stimulationssignal 22 zu erhalten. Mit H(t) für das Signal des Pulszug 35 ergibt sich für das Stimulationssignal 22 folglich S(t) x H(t). Die Stimulationselemente 12 verabreichen das Stimulationssignal 22 dem neuronalen Gewebe und stimulieren die Neuronen mit der pathologisch synchronen neuronalen Aktivität im Zielgebiet 30.

Die Konstruktion des Amplitudenmodulations-Signals für eine lineare zeitverzögerte Feedback-Stimulation ist beschrieben in M. G. Rosenblum, A. S. Pikovsky: Controlling synchronization in an ensemble of globally coupled oscillators. Phys. Rev. Lett. 92, 114102 (2004) und O. V. Popovych, C. Hauptmann, P. A. Tass: Control of neuronal synchrony by nonlinear delayed feedback. Biol. Cybern. 95, 69-85 (2006).

Weiterhin kann aus dem vorverarbeiteten Messsignal x(t) ein nicht-lineares zeitverzögertes Feedback-Modulationssignal S(t) gemäß folgender Gleichung generiert werden:

$$S(t) = IZ^2(t)Z^*(t-\tau), \qquad\qquad (3)$$

wobei für das Signal Z(t) gilt:

$$Z(t) = x(t) + iy(t) \qquad\qquad (4)$$

Z*(t) gibt das komplex Konjugierte von Z(t) an. Das Signal y(t), welches den Imaginärteil des Signals Z(t) darstellt, kann aus dem Signal x(t) durch eine Hilbert-Transformation gewonnen werden. Alternativ kann das Signal y(t) durch Zeitverzögerung aus dem Signal x(t) erzeugt werden. Beispielsweise kann das Signal x(t) um ein Viertel der mittleren Periode T der pathologisch synchronen oszillatorischen neuronalen Aktivität der stimulierten Neuronen verschoben werden: y(t) = x(t - T/4). Die mittlere Periode T der pathologisch synchronen oszillatorischen neuronalen Aktivität der stimulierten Neuronen kann aus dem Messsignal 23 extrahiert werden.

Das Stimulationssignal 22 kann durch Multiplikation des Signals H(t) des Pulszugs 35 mit dem nicht-linearen zeitverzögerten Feedback-Modulationssignal S(t) erzeugt werden. Ferner kann anstelle des komplexen Modulationssignals S(t) nur der Realteil dieses Signals mit dem Signal H(t) multipliziert werden, um das Stimulationssignal 22 zu erhalten. Für den Realteil des nicht-linearen zeitverzögerten Feedback-Modulationssignals S(t) gilt:

$$\mathrm{Re}\big[S(t)\big] = Ix(t-\tau)\big(x^2(t) - y^2(t)\big) + 2Ix(t)y(t)y(t-\tau) \tag{5}$$

Die Konstruktion des Amplitudenmodulations-Signals für eine nicht-lineare zeitverzögerte Feedback-Stimulation ist beschrieben in O. V. Popovych, C. Hauptmann, P. A. Tass: Effective desynchronization by nonlinear delayed feedback. Phys. Rev. Lett. 94, 164102 (2005) und O. V. Popovych, C. Hauptmann, P. A. Tass: Control of neuronal synchrony by nonlinear delayed feedback. Biol. Cybern. 95, 69-85 (2006).

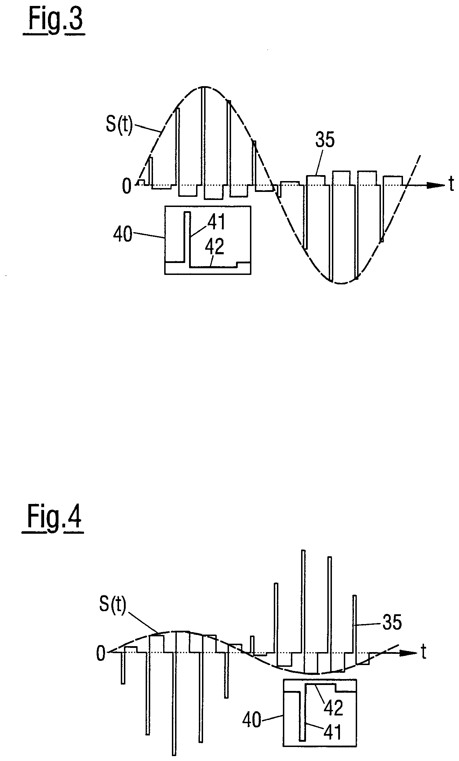

Fig. 3 und 4 zeigen schematisch unterschiedliche elektrische Pulszüge, deren Amplitude mit verschiedenen Feedback-Modulationssignalen S(t) moduliert wurde. Fig. 3 und 4 enthalten jeweils eine separate vergrößerte Darstellung des jeweils verwendeten Einzelpulses 40. Zu beachten ist, dass in Fig. 3 und 4 zur vereinfachten Darstellung die Pausen 43 zwischen den ersten Pulsanteilen 41 und den zweiten Pulsanteilen 42 der Einzelpulse 40 nicht dargestellt sind.

In dem in Fig. 3 gezeigten Pulszug 35 ist der erste Pulsanteil 41 jeweils eine anodische, positive Phase und der zweite Pulsanteil 42 ist eine kathodische, negative Phase. In Fig. 4 sind die Polaritäten der beiden Pulsanteile 41, 42 gegenüber dem Pulszug 35 aus Fig. 3 vertauscht, so dass der erste Pulsanteil 41 eine kathodische Phase und der zweite Pulsanteil 42 eine anodische Phase darstellt.

Wie sich Fig. 3 und 4 entnehmen lässt, sind die Einzelpulse 40 auch nach der Amplitudenmodulation mit dem Feedback-Modulationssignal S(t) derart dimensioniert, dass durch einen Einzelpuls 40 genauso viel Ladung in das Gewebe während der eigentlichen Stimulationsphase eingebracht, wie aus dem Gewebe während der ladungsbalancierenden Stimulationsphase entnommen wird.

Im Zuge der Erfindung wurde die folgende überraschende Beobachtung gemacht: Wird das lineare oder nicht-lineare zeitverzögerte Feedback-Stimulationssignal S(t) zur Amplitudenmodulation eines periodischen Pulszugs, dessen Einzelpulse keine Pause zwischen dem ersten Pulsanteil und dem zweiten Pulsanteil aufweisen, verwandt, so wird keine hinreichend starke Desynchronisation erzielt. Wenn man jedoch wie bei dem in Fig. 2 beispielhaft gezeigten Pulszug 35 eine Pause 43 zwischen dem ersten Pulsanteil 41 und dem zweiten Pulsanteil 42 der Einzelpulse 40 einfügt und die Amplitude des Pulszugs 35 mit dem linearen oder nicht-linearen zeitverzögerten Feedback-Stimulationssignal S(t) moduliert, wird eine voll ausgeprägte Desynchronisation erzielt. Eine deutliche Besserung der Desynchronisation wird schon mit einer Dauer $t_{Pause}$ der Pause 43 von 1 ms erreicht. Bessere Werte für die Dauer $t_{Pause}$ der Pause 43 belaufen sich auf z. B. 5 ms.

Ebenso überraschend ist, dass die Hochfrequenz-Dauerstimulation sowohl mit linearer als auch mit nicht-linearer zeitverzögerter Feedback-Amplitudenmodulation und Einzelpulsen mit Pausen zwischen den Pulsanteilen bzgl. der Abhängigkeit des Desynchronisationseffekts von der Zeitverzögerung vergleichbar sind. D. h., die nicht-lineare Variante ist nicht mehr deutlich überlegen, wie dies bei der herkömmlichen zeitverzögerten Feedback-Stimulation der Fall ist. Somit kann die technisch einfacher realisierbare Variante, also die Hochfrequenz-Dauerstimulation mit linearer zeitverzögerter Feedback-Amplitudenmodulation und Einzelpulsen mit Pausen zwischen den Pulsanteilen verwandt werden. Bei der nicht-linearen Variante werden neben der zeitverzögerten komplex konjugierten Signalkomponente ebenso die insbesondere unverzögerte Signalkomponente verwendet. Im Vergleich hierzu ist es einfacher, die Signalkomponente lediglich zeitlich zu verzögern - wie bei der linearen Variante.

Gemäß einer Ausgestaltung variiert die Steuereinheit 10 die Zeitverzögerung $\tau$ des Feedback-Modulationssignals S(t) gegenüber dem Messsignal 23 oder dem vorverarbeiteten Messsignal x(t). Die Variation der Zeitverzögerung $\tau$ kann insbesondere solange fortgesetzt werden, bis die Synchronisation der stimulierten Neuronen minimal ist oder einen vorgegebenen Schwellwert unterschreitet.

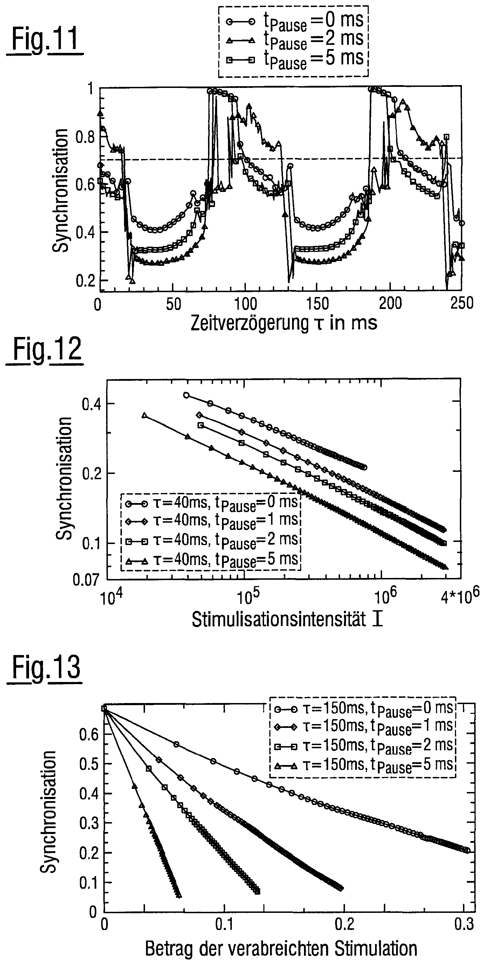

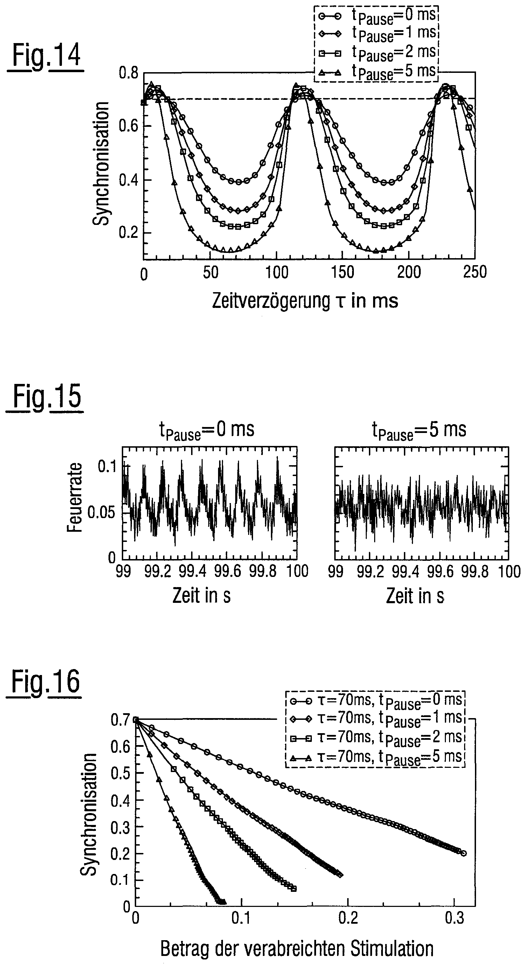

Die optimale Zeitverzögerung $\tau$ kann z. B. im Bereich vom 0,5- bis 2,5-fachen oder vom 1,5- bis 2,5-fachen der mittleren Periode der krankhaften rhythmischen Aktivität der betroffenen Neuronenpopulation liegen. Insbesondere kann die Zeitverzögerung $\tau$ in einem Bereich von 5 ms bis 2 s liegen. Der optimale Wert für die Zeitverzögerung $\tau$ kann auch - z. B. verursacht durch interne Zeitverzögerungen in der Neuronenpopulation bzw. damit interagierender Neuronenpopulationen - stark davon abweichen. Die Abhängigkeit des Stimulationserfolgs von der Zeitverzögerung $\tau$ zeigen die weiter unten erläuterten Figuren 11 und 14. Die zu Bereichen lokaler Minima der Synchronisation gehörenden Zeitverzögerungen wiederholen sich typischerweise nach einem Vielfachen der mittleren Periode. D. h., neben einer

optimalen Zeitverzögerung $\tau_{opt}$ sind auch die Zeitverzögerungen $\tau_{opt} + T_{mittel}$ bzw. $\tau_{opt} + 2T_{mittel}$ für eine ausgeprägte Desynchronisation geeignet, wobei $T_{mittel}$ die mittlere Periode der krankhaften rhythmischen Aktivität der betroffenen Neuronenpopulation bezeichnet. Deswegen kann folgende Kalibrationsprozedur ausgeführt werden: Man bestimmt die mittlere Periode $T_{mittel}$ bzw. nimmt einen dem Fachmann bekannten Wert als Startpunkt. Letzteres ist möglich, da die pathologische oszillatorische Aktivität sich in typischen Frequenzbändern befindet. Man beginnt dann mit einer Zeitverzögerung $\tau = \alpha \cdot T_{mittel}$, wobei $0 < \alpha < 0{,}5$ gilt, z. B. $\alpha = 0{,}4$. Dann lässt man den Parameter $\alpha$ langsam anwachsen, bis sich eine ausgeprägte Desynchronisation einstellt, d. h., die Synchronisation der stimulierten Neuronen ein lokales Minimum erreicht oder einen vorgegebenen Schwellwert unterschreitet. Ein weiteres Anwachsen führt dann zu einer Abnahme der Desynchronisation. Sobald ein Wert für $\alpha$ erreicht ist, der eine optimale Desynchonisation ermöglicht, kann die Intensität, d. h. insbesondere die Amplitude der Stimulation bei dem zuvor ermittelten, festen Wert für $\alpha$ langsam erhöht werden, um eine noch effizientere Desynchronisation erzielen zu können. Die Erhöhung der Intensität kann z. B. fortgesetzt werden, bis die Synchronisation der stimulierten Neuronen ein weiteres lokales Minimum erreicht oder einen weiteren vorgegebenen Schwellwert unterschreitet. Diese Regelvorgänge können automatisiert werden. Es sollte das Feedback des Patienten und/oder Arztes über das Auftreten möglicher Nebenwirkungen bei ansteigender Intensität aber berücksichtigt werden, damit die Prozedur sicher und verträglich ablaufen kann. Da die mittlere Periode $T_{mittel}$ über die Zeit variieren kann, sollte $\alpha$ regelmäßig nachgeregelt werden, um eine optimale Desynchronisation zu ermöglichen.

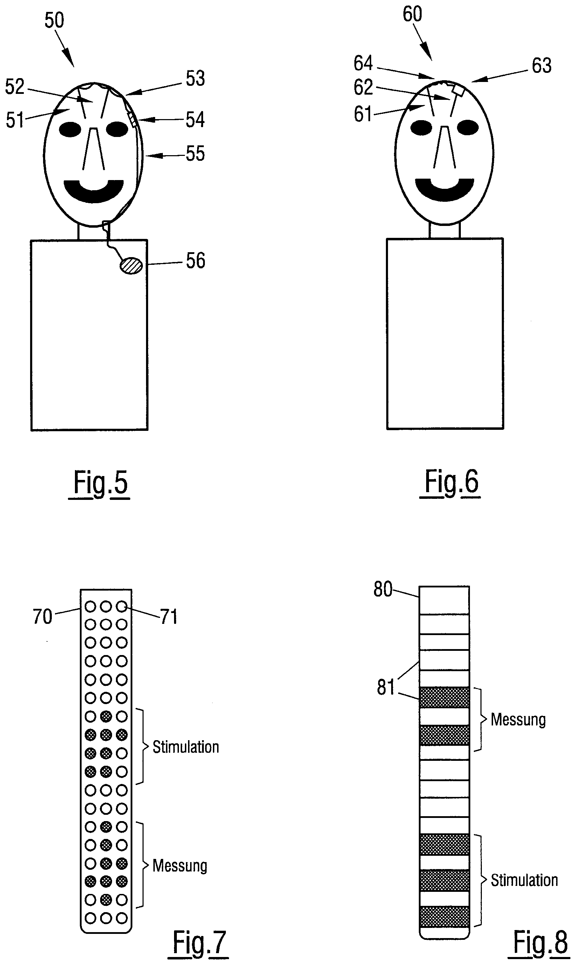

Fig. 5 zeigt schematisch eine Vorrichtung 50 zur invasiven elektrischen Stimulation von Neuronen mit einer krankhaft synchronen und oszillatorischen neuronalen Aktivität gemäß einer Ausführungsform der Erfindung. Die Vorrichtung 50 umfasst zwei tiefe Hirnelektroden 51, 52, die in das Gehirn des Patienten implantiert sind und über Kabel 53 mit einem Konnektor 54 verbunden sind. Der Konnektor 54 wiederum ist über ein Kabel 55 mit einer Steuereinheit 56 verbunden. Die Steuereinheit 56 erzeugt die Stimulationssignale basierend auf den gemessenen Feedback-Signalen. Die Stimulationssignale können für beide Hirnelektroden 51, 52 separat erzeugt werden. Es kann aber auch über eine der beiden Hirnelektroden 51, 52 stimuliert und über die andere Hirnelektrode gemessen werden. Die Vorrichtung 50 kann die Funktion der oben beschriebenen Vorrichtung 1 aufweisen.

Fig. 6 zeigt schematisch eine weitere Vorrichtung 60 zur invasiven elektrischen Stimulation von Neuronen mit einer krankhaft synchronen und oszillatorischen neuronalen Aktivität gemäß einer weiteren Ausführungsform der Erfindung. In gleicher Weise wie die Vorrichtung 50 umfasst die Vorrichtung 60 zwei implantierte tiefe Hirnelektroden 61, 62. Die Vorrichtung 60 umfasst ferner eine im Bohrloch implantierte Steuereinheit 63, die mit der Hirnelektrode 62 direkt verbunden ist. Die Hirnelektrode 61 ist mit der Steuereinheit 63 über ein Kabel 64 verbunden. Die Stimulationssignale können für beide Hirnelektroden 61, 62 separat erzeugt werden. Es kann aber auch über eine der beiden Hirnelektroden 61, 62 stimuliert und über die andere Hirnelektrode gemessen werden. Die Vorrichtung 60 kann die gleichen Funktionen wie die Vorrichtung 1 aufweisen.

Fig. 7 zeigt schematisch eine Vielkanal-Elektrode 70, die als Stimulationseinheit 11 dient und eine Vielzahl von in einem Array angeordneten elektrisch leitfähigen Kontakten bzw. Stimulationskontaktflächen 71 aufweist, welche die Stimulationselemente 12 darstellen. Die Kontakte 71 können einzeln ansteuerbar sein, so dass über jeden Kontakt 71 ein gewünschtes elektrisches Stimulationssignal 22 appliziert werden kann. Beispielsweise kann das Stimulationssignal 22 räumlich gemäß anatomischer und/oder physiologischer Randbedingungen gewichtet über mehrere Kontakte 71 appliziert werden. Ferner können die Kontakte 71 auch zur Messung von neuronaler Aktivität eingesetzt werden. Mess- bzw. Stimulationskontakte 71 sind in Fig. 7 jeweils durch dunkle Scheiben illustriert. Beispielhaft wird über unterschiedliche Gruppen von Kontakten 71 gemessen bzw. stimuliert.

Fig. 8 zeigt schematisch eine Mehrkanal-Elektrode 80, die als Stimulationseinheit 11 dient und eine Vielzahl von ringförmigen elektrisch leitfähigen Kontakten bzw. Stimulationskontaktflächen 81 aufweist, welche die Stimulationselemente 12 darstellen. Über dunkel markierte Kontakte 81 wird hier beispielhaft gemessen oder stimuliert, während über die weiß markierten Kontakte 81 weder gemessen noch stimuliert wird.

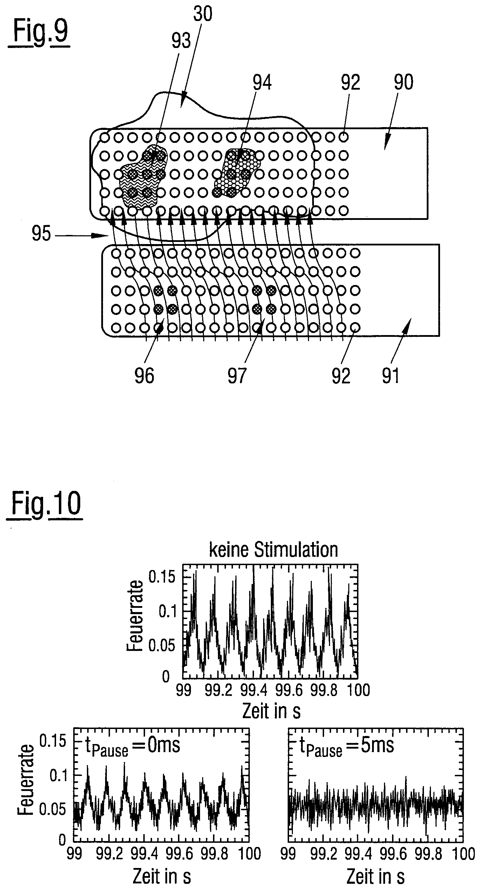

Fig. 9 zeigt schematisch Vielkanal-Elektroden 90, 91, die jeweils eine Vielzahl von in einem Array angeordneten elektrisch leitfähigen Kontakten 92 aufweisen. Mit den Vielkanal-Elektroden 90, 91 werden zwei miteinander interagierende Neuronenpopulationen 93, 94 in dem Zielareal 30 stimuliert. Die Vielkanal-Elektrode 90 ist zur direkten Stimulation der Neuronenpopulationen 93, 94 unmittelbar auf dem Zielareal 30 platziert. Hierdurch können die Somata, Axone und Dendriten der Neuronenpopulationen 93, 94 direkt gereizt werden. In dem vorliegenden Beispiel werden die Neuronenpopulationen 93, 94 über die den Neuronenpopulationen 93, 94 zugeordneten Kontakte 92 mit der dunklen Füllung stimuliert. Jeder der Neuronenpopulationen 93, 94 ist dabei eine Gruppe von Kontakten 92 zugeordnet. Ferner kann über die Vielkanal-Elektrode 90 ein Messsignal, das die neuronale Aktivität der stimulierten Neuronenpopulationen 93, 94 wiedergibt, abgeleitet werden.

Die Vielkanal-Elektrode 91 ist nicht direkt auf dem Zielareal 30 platziert, vielmehr werden afferente Fasern 95, welche zu den Neuronenpopulationen 93, 94 führen und/oder aus ihnen entspringen, gereizt. In dem in Fig. 9 dargestellten Ausführungsbeispiel sind Gruppen 96, 97 aus jeweils mehreren Kontakten 92 gebildet und mit den Gruppen 96, 97 werden über die afferenten Fasern 95 die Neuronenpopulationen 93, 94 indirekt stimuliert. Die Kontakte 92 der Gruppen 96, 97 sind in Fig. 9 mit einer dunklen Füllung dargestellt.

Bei einer kombinierten direkten und indirekten Stimulation kann z. B. eine der Neuronenpopulationen 93, 94 ausschließlich direkt, die andere ausschließlich indirekt mit der oben beschriebenen Hochfrequenz-Dauerstimulation mit linearer sowie nicht-linearer zeitverzögerter Feedback-Amplitudenmodulation stimuliert werden. Prinzipiell kann auch eine gleichzeitige und/oder zeitlich alternierende kombinierte direkte und indirekte Reizung derselben Neuronenpopulation erfolgen.

Bei der direkten und/oder indirekten elektrischen Stimulation können die dem Fachmann bekannten Formen der bipolaren Stimulation zwischen Paaren von Kontakten 92 als auch der unipolaren Stimulation zwischen Kontakten 92 und einer gemeinsamen Masse angewandt werden. Die Messung der Feedback-Signale erfolgt in einer dem Fachmann bekannten Weise über monopolare und/oder bipolare Ableitungen.

Implantierbare Stimulationseinheiten 11 für die optische Stimulation von neuronalem Gewebe sind bekannt. Beispielsweise kann eine Lichtquelle, wie z. B. ein Laser, eine Laserdiode oder eine LED, einen Lichtstrahl erzeugen, der mit Hilfe einer Lichteinkopplung auf die Eingänge eines aus mehreren Lichtleitern bestehenden Faserbündels verteilt wird. Eine Steuereinheit 10 gibt dabei z. B. vor, zu welchem Zeitpunkt ein einzelner Lichtpuls oder ein Zug von Lichtpulsen in welche Faser des Faserbündels eingekoppelt wird. Die Auskopplungspunkte der einzelnen Fasern des Fasernbündels, d. h. die Enden der Fasern, können an verschiedenen Stellen im Zielgebiet 30 im Gehirn und/oder Rückenmark des Patienten liegen. Das Licht stimuliert in diesem Fall unterschiedliche Orte des Zielgebiets 30 in einer durch die Steuereinheit 10 vorgegebenen zeitlichen Abfolge. Es können allerdings auch andere implantierbare Stimulationseinheiten 11 verwendet werden, die sich zur direkten optischen Stimulation von neuronalem Gewebe eignen. Im Fall von optischen Stimulationssignalen 22 wird die Lichtstärke eines Pulszugs mit dem Feedback-Modulationssignals S(t) amplitudenmoduliert.

In den Fig. 10 bis 16 werden die mit der hierin beschriebenen Erfindung erzielbaren Effekte anhand von Simulationsergebnissen veranschaulicht.

Der durch die Erfindung bewirkte Desynchronisationseffekt ist in Fig. 10 anhand der Feuerrate einer Neuronenpopulation gezeigt. Die Feuerrate gibt die relative Anzahl der Neuronen an, die zu einem jeweiligen Zeitpunkt feuern. In den Darstellungen von Fig. 10 ist die Feuerrate der Neuronen gegen die Zeit aufgetragen. In der oberen Darstellung von Fig. 10 ist das rhythmische Feuern der Neuronen der pathologisch synchron aktiven Neuronenpopulation vor der Stimulation gezeigt. Durch eine Hochfrequenz-Dauerstimulation mit einer nicht-linearen zeitverzögerten Feedback-Amplitudenmodulation wird die Synchronisation der Neuronen erheblich reduziert, wie die beiden unteren Darstellungen von Fig. 10 zeigen. Dabei ist eine Stimulation mit Einzelpulsen, die eine Pause $t_{Pause}$ von 5 ms zwischen dem ersten und dem zweiten Pulsanteil aufweisen, einer Stimulation mit Einzelpulsen, deren erste und zweite Pulsanteile direkt aufeinanderfolgen, d. h., für die $t_{Pause}$ = 0 gilt, deutlich überlegen.

In Fig. 11 ist der Grad der Synchronisation einer Neuronenpopulation mit einer krankhaft synchronen und oszillatorischen neuronalen Aktivität in Abhängigkeit von der Zeitverzögerung $\tau$ für eine Hochfrequenz-Dauerstimulation mit einer nicht-linearen zeitverzögerten Feedback-Amplitudenmodulation dargestellt. Die Zeitverzögerung $\tau$ bestimmt, um welche Zeitdauer das Feedback-Modulationssignal S(t) gegenüber dem Messsignal x(t) verzögert ist. Die Simulation wurde für Einzelpulse mit Pausen $t_{Pause}$ von 0, 2 bzw. 5 ms und fester Stimulationsintensität I durchgeführt. Die horizontale gestrichelte Linie in Fig. 11 gibt den Grad der Synchronisation der Neuronenpopulation vor der Stimulation an. Fig. 11 kann entnommen werden, dass sich für bestimmte Teilbereiche der Zeitverzögerung $\tau$ eine deutlich effektivere Desynchronisation der Neuronenpopulation erzielen lässt, wenn zwischen den Pulsanteilen der Einzelpulse eine Pause eingehalten wird.

Der durch die Pause zwischen den Pulsanteilen der Einzelpulse erzielte Desynchronisationseffekt verstärkt sich außerdem bei einer größer werdenden Stimulationsintensität I. Diese Eigenschaft ist in Fig. 12 gezeigt, in welcher der Grad der Synchronisation der stimulierten Neuronenpopulation gegen die Stimulationsintensität I für eine feste Zeitverzögerung $\tau$ von 40 ms und Werte für die Pause $t_{Pause}$ von 0, 1, 2 bzw. 5 ms aufgetragen ist. In dem vorliegenden Beispiel desynchronisiert eine Hochfrequenz-Dauerstimulation mit einer nicht-linearen zeitverzögerten Feedback-Amplitudenmodulation ohne Pause zwischen den Einzelpulsen die Neuronenpopulation nur bis zu einem gewissen Grad. Dieselbe Stimulation mit einer Pause zwischen den Einzelpulsen liefert wesentlich bessere Ergebnisse. Ferner führt eine höhere Stimulationsintensität I zu einer stärken Desynchronisation der Neuronenpopulation.

Die Wirksamkeit der hierin beschriebenen Stimulation ist in Fig. 13 gezeigt, in welcher der Grad der Synchronisation gegen den Betrag der verabreichten Stimulation aufgetragen ist. Der Betrag der verabreichten Stimulation ist gegeben durch die Amplitude des Feedback-Modulationssignals S(t), wie es beispielhaft in Fig. 3 und 4 gezeigt ist. Den in Fig. 13 dargestellten Werten liegt eine Simulation einer Hochfrequenz-Dauerstimulation mit einer nicht-linearen zeitverzögerten Feedback-Amplitudenmodulation mit einer festen Zeitverzögerung $\tau$ von 150 ms und Pausen $t_{Pause}$ von 0, 1, 2 bzw. 5 ms zugrunde. Fig. 13 zeigt, dass eine Stimulation mit einer längeren Pause $t_{Pause}$ einen geringeren Betrag der verabreichten Stimulation benötigt, um den gleichen Desynchronisationseffekt zu erzielen wie eine Stimulation mit einer kürzeren Pause $t_{Pause}$.

Während den in den Fig. 10 bis 13 gezeigten Simulationsergebnissen eine Hochfrequenz-Dauerstimulation mit einer nicht-linearen zeitverzögerten Feedback-Amplitudenmodulation zugrunde lag, zeigen die Fig. 14 bis 16 Simulationsergebnisse einer Hochfrequenz-Dauerstimulation mit einer linearen zeitverzögerten Feedback-Amplitudenmodulation.

Analog zu Fig. 11 zeigt Fig. 14 den Grad der Synchronisation einer Neuronenpopulation mit einer krankhaft synchronen und oszillatorischen neuronalen Aktivität in Abhängigkeit von der Zeitverzögerung $\tau$ für eine Hochfrequenz-Dauerstimulation mit einer linearen zeitverzögerten Feedback-Amplitudenmodulation. Die Simulation wurde für Einzelpulse mit Pausen $t_{Pause}$ von 0, 1, 2 bzw. 5 ms und fester Stimulationsintensität I durchgeführt. Die horizontale gestrichelte Linie in Fig. 14 gibt den Grad der Synchronisation der Neuronenpopulation vor der Stimulation an. Ein Vergleich mit den in Fig. 11 dargestellten Ergebnissen zeigt, dass die lineare zeitverzögerte Feedback-Amplitudenmodulation der nichtlinearen zeitverzögerten Feedback-Amplitudenmodulation für bestimmte Werte der Zeitverzögerung $\tau$ bei ansonsten gleichen Parametern überlegen ist.

In Fig. 15 ist die Feuerrate der Neuronen gegen die Zeit aufgetragen, die durch eine Hochfrequenz-Dauerstimulation mit einer linearen zeitverzögerten Feedback-Amplitudenmodulation und Einzelpulsen ohne bzw. mit Pause $t_{Pause}$ zwischen den Pulsanteilen bewirkt wird. Hier ist genauso wie bei der nicht-linearen zeitverzögerten Feedback-Amplitudenmodulation die Stimulation mit Einzelpulsen, die eine Pause $t_{Pause}$ von 5 ms zwischen dem ersten und dem zweiten Pulsanteil aufweisen, einer Stimulation mit Einzelpulsen, deren erste und zweite Pulsanteile direkt aufeinanderfolgen, deutlich überlegen.

In der Darstellung von Fig. 16 ist der Grad der Synchronisation gegen den Betrag der verabreichten Stimulation aufgetragen, der sich mit einer Hochfrequenz-Dauerstimulation mit einer linearen zeitverzögerten Feedback-Amplitudenmodulation mit einer festen Zeitverzögerung $\tau$ von 70 ms und Pausen $t_{Pause}$ von 0, 1, 2 bzw. 5 ms erzielen lässt. Auch hier führt eine Stimulation mit einer längeren Pause $t_{Pause}$ zu dem gleichen Desynchronisationseffekt wie eine Stimulation mit einer kürzeren Pause $t_{Pause}$, aber einem höheren Betrag der verabreichten Stimulation.

## Patentansprüche

1. Vorrichtung (1) zur Stimulation von Neuronen, umfassend

   - eine in den Körper eines Patienten implantierbare Stimulationseinheit (11) mit mindestens einem Stimulationselement (12) zur Applikation eines Stimulationssignals (22) an das Gewebe des Patienten, um damit Neuronen im Gehirn und/oder Rückenmark des Patienten zu stimulieren,
   - eine Messeinheit (13) zum Aufnehmen eines Messsignals (23), das eine neuronale Aktivität der stimulierten Neuronen wiedergibt, und
   - eine mit der Stimulationseinheit (11) und der Messeinheit (13) gekoppelte Steuereinheit (10), welche
   - aus dem Messsignal (23) ein Modulationssignal erzeugt,
   - die Amplitude eines eine Vielzahl von Einzelpulsen (40) umfassenden Pulszugs (35) mit dem Modulationssignal moduliert, und
   - die Stimulationseinheit (11) derart ansteuert, dass das mindestens eine Stimulationselement (12) den amplitudenmodulierten Pulszug als das Stimulationssignal (22) appliziert, um damit die Neuronen zu stimulieren, wobei
   - die Einzelpulse (40) jeweils einen ersten Pulsanteil (41) und einen dem ersten Pulsanteil (41) nachfolgenden zweiten Pulsanteil (42) aufweisen, und
   - einer von dem ersten Pulsanteil (41) und dem zweiten Pulsanteil (42) Ladung in das Gewebe einbringt und der andere Pulsanteil Ladung aus dem Gewebe entnimmt, **dadurch gekennzeichnet, dass**
   - zwischen dem ersten Pulsanteil (41) und dem zweiten Pulsanteil (42) der Einzelpulse (40) jeweils eine Pause (43) eingehalten wird,
   - das Stimulationssignal (22) dazu ausgelegt ist, bei einer Verabreichung an den Patienten eine krankhaft synchrone und oszillatorische Aktivität der stimulierten Neuronen zu desynchronisieren,
   - das Messsignal (23) die krankhaft synchrone und oszillatorische Aktivität der stimulierten Neuronen wiedergibt, und
   - die Steuereinheit (10) dazu ausgestaltet ist, die Pause (43) zwischen dem ersten Pulsanteil (41) und dem zweiten Pulsanteil (42) zu variieren, bis die Synchronisation der stimulierten Neuronen minimal ist oder einen vorgegebenen Schwellwert unterschreitet.

2. Vorrichtung (1) nach Anspruch 1, wobei die Einzelpulse (40) innerhalb des Pulszugs (35) dauerhaft und/oder periodisch wiederholt werden.

3. Vorrichtung (1) nach Anspruch 1 oder 2, wobei die Einzelpulse (40) innerhalb des Pulszugs (35) periodisch mit einer Frequenz von mindestens 100 Hz wiederholt werden.

**4.** Vorrichtung (1) nach einem der vorhergehenden Ansprüche, wobei die Pause (43) zwischen dem ersten Pulsanteil (41) und dem zweiten Pulsanteil (42) mindestens 1 ms beträgt.

**5.** Vorrichtung (1) nach einem der vorhergehenden Ansprüche, wobei die Steuereinheit (10) das Messsignal (23) durch Verstärkung und/oder Bandpassfilterung vorverarbeitet und aus dem vorverarbeiteten Messsignal das Modulationssignal erzeugt.

**6.** Vorrichtung (1) nach einem der vorhergehenden Ansprüche, wobei die Steuereinheit (10) das Messsignal (23) zeitverzögert und linear verarbeitet, um das Modulationssignal zu erzeugen.

**7.** Vorrichtung (1) nach einem der vorhergehenden Ansprüche, wobei die Steuereinheit (10) das Messsignal (23) zeitverzögert und nicht-linear verarbeitet, um das Modulationssignal zu erzeugen.

**8.** Vorrichtung (1) nach einem der vorhergehenden Ansprüche, wobei die Steuereinheit (10) dazu ausgestaltet ist, die Zeitverzögerung des Messsignals (23) zur Erzeugung des Modulationssignals zu variieren, insbesondere bis die Synchronisation der stimulierten Neuronen ein lokales Minimum erreicht oder einen vorgegebenen Schwellwert unterschreitet.

**9.** Vorrichtung (1) nach Anspruch 8, wobei die Steuereinheit (10) dazu ausgestaltet ist, nach der Variation der Zeitverzögerung die Intensität des Stimulationssignals (22) zu erhöhen, insbesondere bis die Synchronisation der stimulierten Neuronen ein weiteres lokales Minimum erreicht oder einen weiteren vorgegebenen Schwellwert unterschreitet.

**10.** Vorrichtung (1) nach einem der vorhergehenden Ansprüche, wobei der Pulszug (35) ein elektrischer Pulszug oder ein optischer Pulszug ist.

**11.** Vorrichtung (1) nach einem der vorhergehenden Ansprüche, wobei die ersten und zweiten Pulsanteile (41, 42) derart dimensioniert sind, dass der eine Pulsanteil genauso viel Ladung in das Gewebe einbringt, wie der andere Pulsanteil dem Gewebe entnimmt.

**12.** Vorrichtung (1) nach einem der vorhergehenden Ansprüche, wobei die Pause (43) zwischen dem ersten Pulsanteil (41) und dem zweiten Pulsanteil (42) der Einzelpulse (40) kleiner ist als die Pause zwischen dem zweiten Pulsanteil (42) eines Einzelpulses (40) und dem ersten Pulsanteil (41) des im Pulszug (35) direkt nachfolgenden Einzelpulses (40).

**13.** Software zum Ausführen in der Steuereinheit (10) der Vorrichtung (1) nach einem der vorhergehenden Ansprüche, wobei die Software

   - Steuersignale zum Ansteuern einer in den Körper eines Patienten implantierten Stimulationseinheit (11) mit mindestens einem Stimulationselement (12) erzeugt, wobei
   - das mindestens eine Stimulationselement (12) ein Stimulationssignal (22) an das Gewebe des Patienten appliziert, um damit Neuronen im Gehirn und/oder Rückenmark des Patienten zu stimulieren,
   - ein Messsignal (23) aufgenommen wird, das eine neuronale Aktivität der stimulierten Neuronen wiedergibt,
   - aus dem Messsignal (23) ein Modulationssignal erzeugt wird,
   - die Amplitude eines eine Vielzahl von Einzelpulsen (40) umfassenden Pulszugs (35) mit dem Modulationssignal moduliert wird, und
   - das mindestens eine Stimulationselement (12) den amplitudenmodulierten Pulszug als das Stimulationssignal (22) appliziert, um damit die Neuronen zu stimulieren, wobei
   - die Einzelpulse (40) jeweils einen ersten Pulsanteil (41) und einen dem ersten Pulsanteil (41) nachfolgenden zweiten Pulsanteil (42) aufweisen,
   - einer von dem ersten Pulsanteil (41) und dem zweiten Pulsanteil (42) Ladung in das Gewebe einbringt und der andere Pulsanteil Ladung aus dem Gewebe entnimmt,
   - zwischen dem ersten Pulsanteil (41) und dem zweiten Pulsanteil (42) der Einzelpulse (40) jeweils eine Pause (43) eingehalten wird,
   - das Stimulationssignal (22) dazu ausgelegt ist, bei einer Verabreichung an den Patienten eine krankhaft synchrone und oszillatorische Aktivität der stimulierten Neuronen zu desynchronisieren,
   - das Messsignal (23) die krankhaft synchrone und oszillatorische Aktivität der stimulierten Neuronen wiedergibt, und

- die Pause (43) zwischen dem ersten Pulsanteil (41) und dem zweiten Pulsanteil (42) variiert wird, bis die Synchronisation der stimulierten Neuronen minimal ist oder einen vorgegebenen Schwellwert unterschreitet.

**Claims**

1. An apparatus (1) for stimulating neurons, comprising

   - a stimulation unit (11) implantable into a patient's body, said stimulation unit (11) having at least one stimulation element (12) for applying a stimulation signal (22) to the patient's tissue in order to stimulate therewith neurons in the patient's brain and/or spinal cord,
   - a measurement unit (13) for recording a measurement signal (23) that represents a neuronal activity of the stimulated neurons, and
   - a control unit (10) coupled with the stimulation unit (11) and the measurement unit (13), which
   - creates a modulation signal from the measurement signal (23),
   - modulates the amplitude of a pulse train (35) comprising a plurality of individual pulses (40) with the modulation signal, and
   - controls the stimulation unit (11) such that the at least one stimulation element (12) applies the amplitude-modulated pulse train as the stimulation signal (22) in order to stimulate therewith the neurons, wherein
   - each of the individual pulses (40) comprises a first pulse portion (41) and a second pulse portion (42) following the first pulse portion (41), and
   - one of the first pulse portion (41) and the second pulse portion (42) imparts charge to the tissue and the other pulse portion takes charge from the tissue, **characterized in that**
   - a break (43) is taken between each first pulse portion (41) and each second pulse portion (42) of the individual pulses (40),
   - the stimulation signal (22) is configured to desynchronize an abnormally synchronous and oscillatory activity of the stimulated neurons when being applied to the patient,
   - the measurement signal (23) represents the abnormally synchronous and oscillatory activity of the stimulated neurons, and
   - the control unit (10) is configured to vary the break (43) between the first pulse portion (41) and the second pulse portion (42) until synchronization of the stimulated neurons is at a minimum or falls below a predetermined threshold value.

2. The apparatus (1) according to claim 1, wherein the individual pulses (40) are permanently and/or periodically repeated within the pulse train (35).

3. The apparatus (1) according to claim 1 or 2, wherein the individual pulses (40) are periodically repeated at a frequency of at least 100 Hz within the pulse train (35).

4. The apparatus (1) according to any one of the preceding claims, wherein the break (43) between the first pulse portion (41) and the second pulse portion (42) is at least 1 ms.

5. The apparatus (1) according to any one of the preceding claims, wherein the control unit (10) preprocesses the measurement signal (23) by means of amplification and/or a band pass filter and creates the modulation signal from the preprocessed measurement signal.

6. The apparatus (1) according to any one of the preceding claims, wherein the control unit (10) processes the measurement signal (23) in a time-delayed and linear manner in order to create the modulation signal.

7. The apparatus (1) according to any one of the preceding claims, wherein the control unit (10) processes the measurement signal (23) in a time-delayed and nonlinear manner in order to create the modulation signal.

8. The apparatus (1) according to any one of the preceding claims, wherein the control unit (10) is configured to vary the time delay of the measurement signal (23) for creating the modulation signal, particularly until the synchronization of the stimulated neurons reaches a local minimum or falls below a predetermined threshold value.

9. The apparatus (1) according to claim 8, wherein the control unit (10) is configured to increase the intensity of the stimulation signal (22) after varying the time delay, particularly until the synchronization of the stimulated neurons reaches another local minimum or falls below another predetermined threshold value.

10. The apparatus (1) according to any one of the preceding claims, wherein the pulse train (35) is an electric pulse train or an optical pulse train.

11. The apparatus (1) according to any one of the preceding claims, wherein the first and second pulse portions (41, 42) are dimensioned in such a way that said one pulse portion imparts the same amount of charge to the tissue as the other pulse portion takes from the tissue.

12. The apparatus (1) according to any one of the preceding claims, wherein the break (43) between the first pulse portion (41) and the second pulse portion (42) of the individual pulses (40) is smaller than the break between the second pulse portion (42) of an individual pulse (40) and the first pulse portion (41) of the individual pulse (40) following directly in the pulse train (35).

13. A software for execution in the control unit (10) of the apparatus (1) according to any one of the preceding claims, wherein the software

- creates control signals for controlling a stimulation unit (11) implanted in a patient's body, said stimulation unit (11) having at least one stimulation element (12), wherein
- the at least one stimulation element (12) applies a stimulation signal (22) to the patient's tissue in order to stimulate neurons in the patient's brain and/or spinal cord,
- a measurement signal (23) which represents a neuronal activity of the stimulated neurons is recorded,
- a modulation signal is created from the measurement signal (23),
- the amplitude of a pulse train (35) comprising a plurality of individual pulses (40) is modulated with the modulation signal, and
- the at least one stimulation element (12) applies the amplitude-modulated pulse train as the stimulation signal (22) in order to stimulate therewith the neurons, wherein
- each of the individual pulses (40) comprises a first pulse portion (41) and a second pulse portion (42) following the first pulse portion (41),
- one of the first pulse portion (41) and the second pulse portion (42) imparts charge to the tissue and the other pulse portion takes charge from the tissue,
- a break (43) is taken between each first pulse portion (41) and each second pulse portion (42) of the individual pulses (40),
- the stimulation signal (22) is configured to desynchronize an abnormally synchronous and oscillatory activity of the stimulated neurons when being applied to the patient,
- the measurement signal (23) represents the abnormally synchronous and oscillatory activity of the stimulated neurons, and
- the break (43) between the first pulse portion (41) and the second pulse portion (42) is being varied until synchronization of the stimulated neurons is at a minimum or falls below a predetermined threshold value.

## Revendications

1. Dispositif (1) de stimulation de neurones, comprenant

- une unité de stimulation (11) pouvant être implantée dans le corps d'un patient, avec au moins un élément de stimulation (12) pour l'application d'un signal de stimulation (22) au tissu du patient, afin de stimuler ainsi des neurones dans le cerveau et/ou la moelle épinière du patient,
- une unité de mesure (13) destinée à enregistrer un signal de mesure (23), qui représente une activité neuronale des neurones stimulés, et
- une unité de commande (10) couplée à l'unité de stimulation (11) et à l'unité de mesure (13), laquelle
- produit à partir du signal de mesure (23) un signal de modulation,
- module l'amplitude d'un train d'impulsions (35) comprenant une pluralité d'impulsions individuelles (40) avec le signal de modulation, et
- commande l'unité de stimulation (11) de telle sorte que l'au moins un élément de stimulation (12) applique le train d'impulsions modulé en amplitude en tant que signal de stimulation (22), afin de stimuler ainsi les neurones, dans lequel
- les impulsions individuelles (40) présentent respectivement une première part d'impulsion (41) et une deuxième part d'impulsion (42) suivant la première part d'impulsion (41), et

- une de la première part d'impulsion (41) et de la deuxième part d'impulsion (42) introduit une charge dans le tissu et l'autre part d'impulsion retire la charge du tissu, **caractérisé en ce que**

- respectivement une pause (43) est respectée entre la première part d'impulsion (41) et la deuxième part d'impulsion (42) des impulsions individuelles (40),

- le signal de stimulation (22) est conçu pour désynchroniser une activité pathologiquement synchrone et oscillatoire des neurones stimulés lorsqu'il est administré au patient,

- le signal de mesure (23) représente l'activité pathologiquement synchrone et oscillatoire des neurones stimulés, et

- l'unité de commande (10) est conçue pour modifier la pause (43) entre la première part d'impulsion (41) et la deuxième part d'impulsion (42), jusqu'à ce que la synchronisation des neurones stimulés soit minimale ou passe au-dessous d'une valeur seuil prédéfinie.

2.  Dispositif (1) selon la revendication 1, dans lequel les impulsions individuelles (40) sont répétées en permanence et/ou périodiquement à l'intérieur du train d'impulsions (35).

3.  Dispositif (1) selon la revendication 1 ou 2, dans lequel les impulsions individuelles (40) sont répétées périodiquement à une fréquence d'au moins 100 Hz à l'intérieur du train d'impulsions (35).

4.  Dispositif (1) selon l'une quelconque des revendications précédentes, dans lequel la pause (43) entre la première part d'impulsion (41) et la deuxième part d'impulsion (42) atteint au moins 1 ms.

5.  Dispositif (1) selon l'une quelconque des revendications précédentes, dans lequel l'unité de commande (10) prétraite le signal de mesure (23) par amplification et/ou filtrage passe-bande et produit le signal de modulation à partir du signal de mesure prétraité.

6.  Dispositif (1) selon l'une quelconque des revendications précédentes, dans lequel l'unité de commande (10) retarde et traite linéairement le signal de mesure (23), afin de produire le signal de modulation.

7.  Dispositif (1) selon l'une quelconque des revendications précédentes, dans lequel l'unité de commande (10) retarde et traite non linéairement le signal de mesure (23), afin de produire le signal de modulation.

8.  Dispositif (1) selon l'une quelconque des revendications précédentes, dans lequel l'unité de commande (10) est conçue pour modifier le retard du signal de mesure (23) pour produire le signal de modulation, en particulier jusqu'à ce que la synchronisation des neurones stimulés atteigne un minimum local ou passe au-dessous d'une valeur seuil prédéfinie.

9.  Dispositif (1) selon la revendication 8, dans lequel l'unité de commande (10) est conçue pour augmenter l'intensité du signal de stimulation (22) après la variation du retard, en particulier jusqu'à ce que la synchronisation des neurones stimulés atteigne un autre minimum local ou passe au-dessous d'une autre valeur seuil prédéfinie.

10. Dispositif (1) selon l'une quelconque des revendications précédentes, dans lequel le train d'impulsions (35) est un train d'impulsions électriques ou un train d'impulsions optiques.

11. Dispositif (1) selon l'une quelconque des revendications précédentes, dans lequel les première et deuxième parts d'impulsion (41, 42) sont dimensionnées de telle sorte que l'une des parts d'impulsion introduit dans le tissu autant de charge que l'autre part d'impulsion en extrait du tissu.

12. Dispositif (1) selon l'une quelconque des revendications précédentes, dans lequel la pause (43) entre la première part d'impulsion (41) et la deuxième part d'impulsion (42) des impulsions individuelles (40) est inférieure à la pause entre la deuxième part d'impulsion (42) d'une impulsion individuelle (40) et la première part d'impulsion (41) de l'impulsion individuelle (40) directement consécutive dans le train d'impulsions (35).

13. Logiciel à exécuter dans l'unité de commande (10) du dispositif (1) selon l'une quelconque des revendications précédentes, dans lequel le logiciel

- produit des signaux de commande destinés à commander une unité de stimulation (11) implantée dans le corps d'un patient avec au moins un élément de stimulation (12), dans lequel

- l'au moins un élément de stimulation (12) applique un signal de stimulation (22) au tissu du patient, afin de stimuler ainsi des neurones dans le cerveau et/ou la moelle épinière du patient,
- un signal de mesure (23) est enregistré, qui représente une activité neuronale des neurones stimulés,
- un signal de modulation est produit à partir du signal de mesure (23),
- l'amplitude d'un train d'impulsions (35) comprenant une pluralité d'impulsions individuelles (40) est modulée avec le signal de mesure, et
- l'au moins un élément de stimulation (12) applique le train d'impulsions modulé en amplitude en tant que signal de stimulation (22), afin de stimuler ainsi les neurones, dans lequel
- les impulsions individuelles (40) présentent respectivement une première part d'impulsion (41) et une deuxième part d'impulsion (42) suivant la première part d'impulsion (41),
- une de la première part d'impulsion (41) et de la deuxième part d'impulsion (42) introduit une charge dans le tissu et l'autre part d'impulsion extrait la charge du tissu,
- respectivement une pause (43) est respectée entre la première part d'impulsion (41) et la deuxième part d'impulsion (42) des impulsions individuelles (40),
- le signal de stimulation (22) est conçu pour désynchroniser une activité pathologiquement synchrone et oscillatoire des neurones stimulés lorsqu'il est administré au patient,
- le signal de mesure (23) représente l'activité pathologiquement synchrone et oscillatoire des neurones stimulés, et
- la pause (43) entre la première part d'impulsion (41) et la deuxième part d'impulsion (42) est modifiée jusqu'à ce que la synchronisation des neurones stimulés soit minimale ou passe au-dessous d'une valeur seuil prédéfinie.

## Fig.1

## Fig.2

## Fig.3

S(t)

0

35

40

41

42

t

## Fig.4

S(t)

0

35

42

41

40

t

Fig.5

Fig.6

Fig.7

Fig.8

## Fig.9

## Fig.10

## Fig.11

Legend:
- $t_{Pause} = 0$ ms
- $t_{Pause} = 2$ ms
- $t_{Pause} = 5$ ms

Y-axis: Synchronisation
X-axis: Zeitverzögerung $\tau$ in ms

## Fig.12

Legend:
- $\tau = 40$ms, $t_{Pause} = 0$ ms
- $\tau = 40$ms, $t_{Pause} = 1$ ms
- $\tau = 40$ms, $t_{Pause} = 2$ ms
- $\tau = 40$ms, $t_{Pause} = 5$ ms

Y-axis: Synchronisation
X-axis: Stimulisationsintensität $I$

## Fig.13

Legend:
- $\tau = 150$ms, $t_{Pause} = 0$ ms
- $\tau = 150$ms, $t_{Pause} = 1$ ms
- $\tau = 150$ms, $t_{Pause} = 2$ ms
- $\tau = 150$ms, $t_{Pause} = 5$ ms

Y-axis: Synchronisation
X-axis: Betrag der verabreichten Stimulation

## Fig.14

Legend:
- ⊙—⊙ $t_{Pause}=0$ ms
- ◇—◇ $t_{Pause}=1$ ms
- ▱—▱ $t_{Pause}=2$ ms
- △—△ $t_{Pause}=5$ ms

Y-axis: Synchronisation
X-axis: Zeitverzögerung $\tau$ in ms

## Fig.15

$t_{Pause}=0$ ms          $t_{Pause}=5$ ms

Y-axis: Feuerrate
X-axis: Zeit in s

## Fig.16

Legend:
- ⊙—⊙ $\tau=70$ms, $t_{Pause}=0$ ms
- ◇—◇ $\tau=70$ms, $t_{Pause}=1$ ms
- ▱—▱ $\tau=70$ms, $t_{Pause}=2$ ms
- △—△ $\tau=70$ms, $t_{Pause}=5$ ms

Y-axis: Synchronisation
X-axis: Betrag der verabreichten Stimulation

21

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20100217355 A1

### In der Beschreibung aufgeführte Nicht-Patentliteratur

- **P. TEMPERLI ; J. GHIKA ; J.-G. VILLEMURE ; P. BURKHARD ; J. BOGOUSSLAVSKY ; F. VINGERHOETS.** How do parkinsonian signs return after discontinuation of subthalamic DBS?. *Neurology,* 2003, vol. 60, 78
- **P. A. TASS ; L. QIN ; C. HAUPTMANN ; S. DOVEROS ; E. BEZARD ; T. BORAUD ; W. G. MEISSNER.** Coordinated reset neuromodulation has sustained after-effects in parkinsonian monkeys. *Annals of Neurology,* 2012, vol. 72, 816-820
- **I. ADAMCHIC ; C. HAUPTMANN ; U. B. BARNIKOL ; N. PAWELCYK ; O.V. POPOVYCH ; T. BARNIKOL ; A. SILCHENKO ; J. VOLKMANN ; G. DEUSCHL ; W. MEISSNER.** Coordinated Reset Has Lasting Aftereffects in Patients with Parkinson's Disease. *Movement Disorders,* 2014, vol. 29, 1679
- **M. G. ROSENBLUM ; A. S. PIKOVSKY.** Controlling synchronization in an ensemble of globally coupled oscillators. *Physical Review Letters,* 2004, vol. 92, 114102
- **O. V. POPOVYCH ; C. HAUPTMANN ; P. A. TASS.** Effective Desynchronization by Nonlinear Delayed Feedback. *Physical Review Letters,* 2005, vol. 94, 164102

- **S. B. BRUMMER ; M. TURNER, M.** Electrical stimulation of the nervous system: the principle of safe charge injection with noble metal electrodes. *Bioelectrochem. Bioenerg,* 1975, vol. 2, 13
- **S. B. BRUMMER ; L. S. ROBBLEE ; F. T. HAMBRECHT.** Criteria for selecting electrodes for electrical stimulation: theoretical and practical considerations. *Ann. N. Y. Acad. Sci.,* 1983, vol. 405, 159
- **D. HARNACK ; C. WINTER ; W. MEISSNER ; T. REUM ; A. KUPSCH ; R. MORGENSTERN.** The effects of electrode material, charge density and stimulation duration on the safety of high-frequency stimulation of the subthalamic nucleus in rats. *J. Neurosci. Methods,* 2004, vol. 138, 207
- **M. G. ROSENBLUM ; A. S. PIKOVSKY.** Controlling synchronization in an ensemble of globally coupled oscillators. *Phys. Rev. Lett.,* 2004, vol. 92, 114102
- **O. V. POPOVYCH ; C. HAUPTMANN ; P. A. TASS.** Control of neuronal synchrony by nonlinear delayed feedback. *Biol. Cybern.,* 2006, vol. 95, 69-85
- **O. V. POPOVYCH ; C. HAUPTMANN ; P. A. TASS.** Effective desynchronization by nonlinear delayed feedback. *Phys. Rev. Lett.,* 2005, vol. 94, 164102